# EUROPEAN PATENT SPECIFICATION

(11) **EP 1 322 781 B1**
(45) Date of publication and mention of the grant of the patent: **21.02.2007**
(21) Application number: 01961486.6
(22) Date of filing: 01.08.2001
(51) Int. Cl.: C12Q 1/68

(54) **Method and kit for determining human geographic or population origin**
Verfahren und Kit zur Bestimmung der geographischen bzw. populationsmässigen Herkunft von Menschen
Procédé et trousse permettant de déterminer l'origine géographique ou démographique d'un être humain

(30) Priority: 15.09.2000 SE 0003286
(43) Date of publication of application: 02.07.2003
(73) Proprietor: Gyllensten, Ulf, 756 47 Uppsala (SE); Ingman, Max, 752 40 Uppasala (SE); Allen, Marie, 756 47 Uppasala (SE); Andreasson, Hanna, 756 47 Uppsala (SE)
(72) Inventor: Gyllensten, Ulf, 756 47 Uppsala (SE); Ingman, Max, 752 40 Uppasala (SE); Allen, Marie, 756 47 Uppasala (SE); Andreasson, Hanna, 756 47 Uppsala (SE)
(74) Representative: Hagström, Lena
(86) International application number: PCT/SE2001/001691
(87) International publication number: WO 2002/022873

(56) References cited:
- EP-A2- 0 812 922
- SAARA FINNILA ET AL.: 'Phylogenetic network of the mtDNA haplogroup U in Northern Finland based on sequence analysis of the complete coding region by conformation-sensitive gel electrophoresis' AM. J. HUM. GENET. vol. 66, 2000, pages 1017 - 1026, XP002963131
- ANNA DI RIENZO ET AL.: 'Branching pattern in the evolutionary tree for human mitochondrial DNA' PROC. NATL. ACAD. SCI. USA vol. 88, March 1991, pages 1597 - 1601, XP002963129

## Description

### Field of invention

The present invention is within the fields of human origins, medicine and evolutionary biology. More precisely, the invention relates to a method and kit for determining the geographic or population origin of a human based on mitochondrial DNA sequences and specifically to the use of mitochondrial DNA variants (polymorphisms) from the complete human mitochondrial genome. This information is employed in the comparison of biological samples with samples of known origin or with a database of mitochondrial genome sequences.

### Background of the invention

Each individual, with the exception of identical twins, has a unique genetic constitution. Therefore, identification of the genetic origin can, in principle, be based on a comparison of the genetic material between e.g. a sample of unknown human origin and reference samples of known origin. Such an analysis is of relevance in maternity and paternity investigations, in immigration cases where the familial relationships is disputed, in medical and evolutionary biology research.

The genetic material (DNA) in humans is found in the cell nucleus (containing over 99% of the material) and the mitochondrion (with less than 1%). The DNA in the nucleus is inherited with 50% from each parent, while the DNA in the mitochondrion (called the mtDNA) is derived solely from the mother (a process denoted maternal inheritance). Several characteristics of the mitochondrial genome makes it different from that in the nucleus. The high mtDNA copy number per cell (1,000 - 10,000 copies/cell) allows for the analysis of materials with limited amounts of, or partly degraded, DNA (Bodenhagen and Clayton, 1974). The higher nucleotide substitution rate of mtDNA relative to most nuclear genes also increases the potential for individual identification (Brown et al. 1982). Finally, mtDNA is inherited uniparentally, through the maternal parent (Hutchinson et al. 1974). Since the mtDNA of siblings and close maternal relatives are expected to be identical, with the exception of new mutations, individuals may be assigned to a maternal lineage. Due to the haploid, or clonal nature, of mtDNA, jumping-PCR (Pääbo et al. 1989), which commonly occurs in degraded DNA, does not cause erroneous results. By contrast, in analysis of nuclear loci, jumping PCR may result in the presence of chimeric sequences that complicate the interpretation of the allelic phase of polymorphisms,

Studies of human origins have been based on analyses of small segments of either nuclear DNA or mtDNA. The studies of mtDNA have been confined to a small segment of the mtDNA genome, denoted the D-loop (Higuchi et al. 1988, Wilson et al. 1993, Ginther et al. 1992, Hagerberg and Sykes 1989, Stoneking et al. 1991, Holland et al. 1993, Handt et al. 1994, Gill et al. 1994, Allen et al. 1998). No studies including the entire mtDNA for the purpose of determining the maternal lineage and deducing the geographic or population origin have been described. Analysis of the D-loop region have been complicated by the extreme variation in substitution rate between different nucleotide sites (necessitating the exclusion of some sites from the analysis) and the relatively high rate of new mutations (potentially resulting in false inclusions/exclusions). Different tissues of an individual may also show differences in the D-loop mtDNA, rendering comparisons between samples from maternal relatives but of different tissue type ambiguous.

Studies have been performed to estimate probability by which the major ethnic group (or geographic region) of an individual can be estimated based on D-loop sequences (Allen et al. 1995), Using D-loop sequences alone is it impossibile to identify the geographic or population origin of an individual.

According to one study, a human mitochondrial DNA (mtDNA) standard reference material (SRM2392) will provide quality control when mtDNA is sequenced for forensic identifications, medical diagnosis, or mutation detection. Fifty-eight primer sets allow any area or the entire mtDNA to be amplified and sequenced for this purpose (Levin et al 1999).

In another study, eighty-eight types of mtDNA were found by sequencing the most variable part of the control region from 117 Caucasians. The mtDNA sequences were used in order to analyse the branching pattern in the evolutionary tree for humans (Di Rienzo et al 1991).

In a further study analysis of selected polymorphisms in mitochondria is described. Samples from 22 Finns belonging to haplogroup U were tested. Sixty-three PCR fragments covering the coding region were analyzed by conformation-sensitive gel electrophoresis. The phylogenetic network of the 22 coding-regon sequences constituted a tree free from homoplasy, and provided several previously unidentified polymorphisms characterizing subgroups of U (Finnilä et al 2000).

In a further prior art document sequencing of the complete mitochondrial genome of several individuals is described and a large set of polymorphisms is identified (EP-A2-0812922).

Studies of the human mitochondrial molecule have also been carried out through RFLP analysis, providing data on some DNA variants outside the D-loop, These analyses were carried out to study the evolutionary history of the human species, or to identify mutations causing mitochondrial disease and were not performed for the purpose of determining the deducing the geographic and population origin of an individual.

### Summary of the invention

The present invention provides a method for determining the geographic and population origin of an individual based on analysis of biological samples. According to the present invention the analysis is based on an analysis of the entire mitochondrial DNA (mtDNA) outside the D-loop and comparison of the sample under investigation with that of known origin or with a database of compete mitochondrial genome sequences.

In a first aspect the present invention relates to a method for determining the geographic and population origin of a human comprising the following steps:
a) determining the complete nucleic acid sequence of the mitochondrial genome outside the D-loop including polymorphic sites in a sample from a human subject who's origin or identity is studied; and
b) relating the information from step a) to mitochondrial nucleic acid sequence information of known origin.

The body sample referred to above can be derived from body fluid or tissue.

The known information in step b) may be derived from human subjects of known identity (reference subjects). Alternatively, the known information in step b) is derived from a database of the nucleic acid sequence information the complete mitochondrial genome of humans of diverse origin. This may be appropriate in cases where samples from maternal relatives are not available, or when otherwise considered informative.

The mitochondrial DNA sequence information is from the entire mtDNA molecule (about 16.500 nucleotides). The information is obtained from a fragment hereof, not including the D-loop. The analysis of the complete mtDNA sequence or fragments thereof, may be carried out using existing technology for DNA sequencing. Analysis of the genetic markers may be based on DNA hybridisation assays (such as ASO hybridisation, DNA microchip, padlock), enzymatic cleavage assays (OLA, Taqman), enzymatic extension assays (minisequencing, pyrosequencing) or other suitable techniques for DNA typing.

In a second aspect, the present invention provides a kit for determining the geographic and population origin of a human, comprising means for sequence analysis of the complete human mitochondrial DNA outside the D-loop of unknown origin and mitochondrial DNA sequence information of known origin. This sequence information can be provided as a leaflet with printed sequence information or as a printed reference to a database. The means for sequence analysis can be any means used in the known DNA sequencing procedures described above. The sequence of unknown origin is compared with the known sequences or a database with complete mitochondrial genomes and conclusions about geographic and population origin can be made.

The kit comprising means for analysis of polymorphic sites, located outside the D-loop is disclosed. Polymorphic sites useful for the purposes of the present invention are listed in Table 1. The D-loop is numbered from 16028 to 577 (i.e. 16028 to 16569 and 1-577).

In an alternative embodiment, the present invention provides a specific set of laboratory reagents and conditions (protocol) for determining the type of nucleotide at a selected set of the polymorphic sites in the mitochondrial genome, based on using the pyrosequencing method.

### Detailed description of the invention

The invention will now be described more closely in association with the accompanying drawings, in which

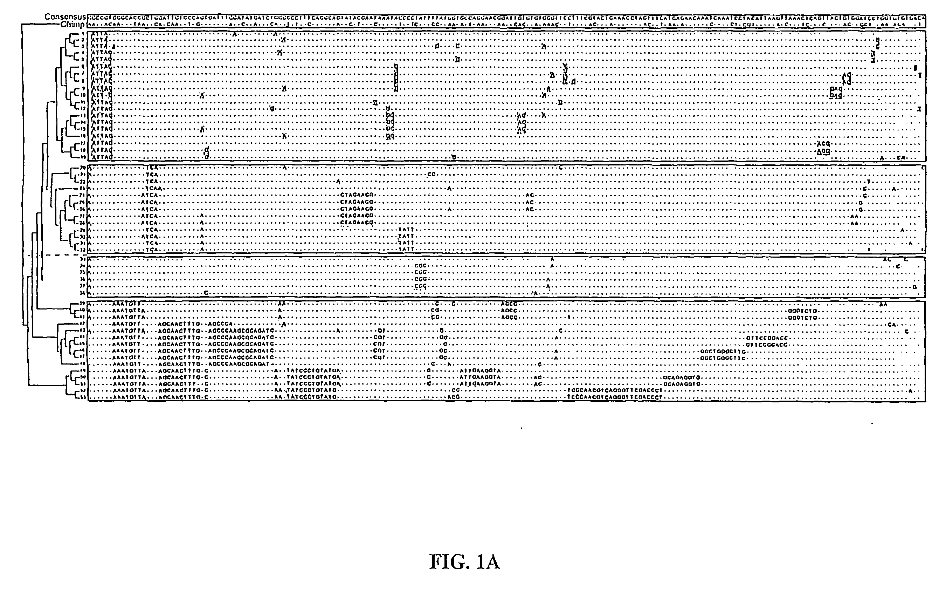

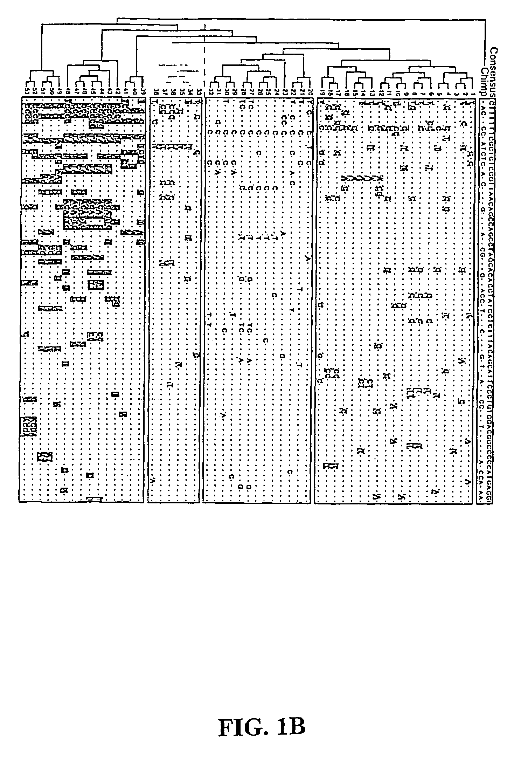

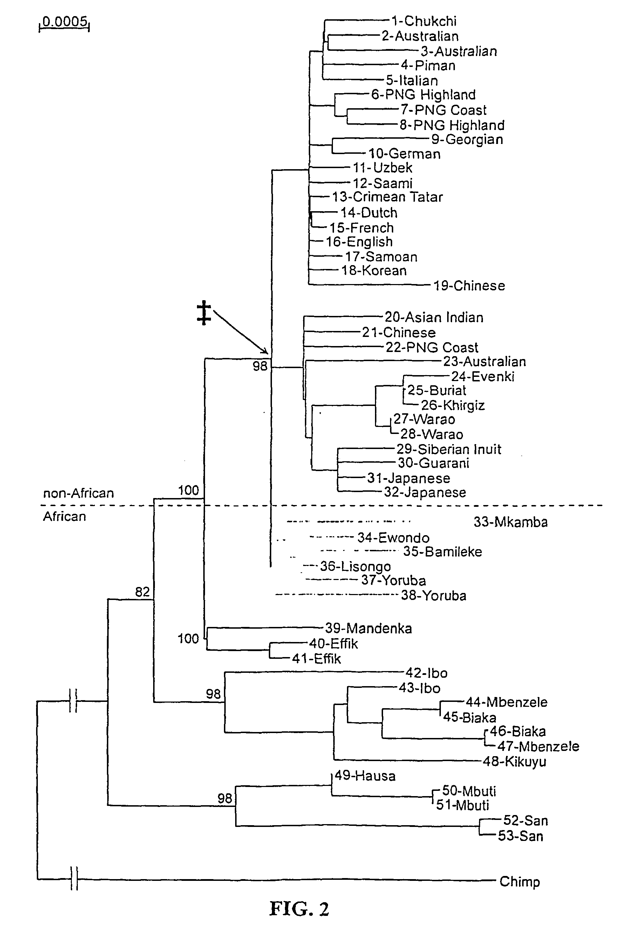

Figure 1 represents data matrices showing all informative nucleotide positions in 53 of the 124 individuals for which the complete mitochondrial genome has been determined, in decreasing order of frequency, in: a) the whole mtDNA genome, excluding the D-loop and; b) the D-loop. The trees on the left are cladograms with the same topology and numbering of individuals as the tree in Figure 2. Individuals of African decent are found exclusively below the dashed line and non-Africans above. The four major groups of sequences are boxed in blocks. The blocks denote groups of nucleotides identical in several sequences.

Figure 2 shows a Neighbor-Joining phylogram based on complete mtDNA genome sequences (but excluding the D-loop) of 53 of the 124 individuals examined, constructed using PAUP*4.0 Beta (Sinauer Associates) and bootstrapped with 1000 replicates (bootstrap values shown on nodes). The population origin of the individual is given at the twigs. The branches are shown block-wise as in Fig 1. Individuals of African descent are found exclusively below the dashed line and non-Africans above. The node marked '‡' refers to the MRCA of the youngest clade containing both African and non-African individuals.

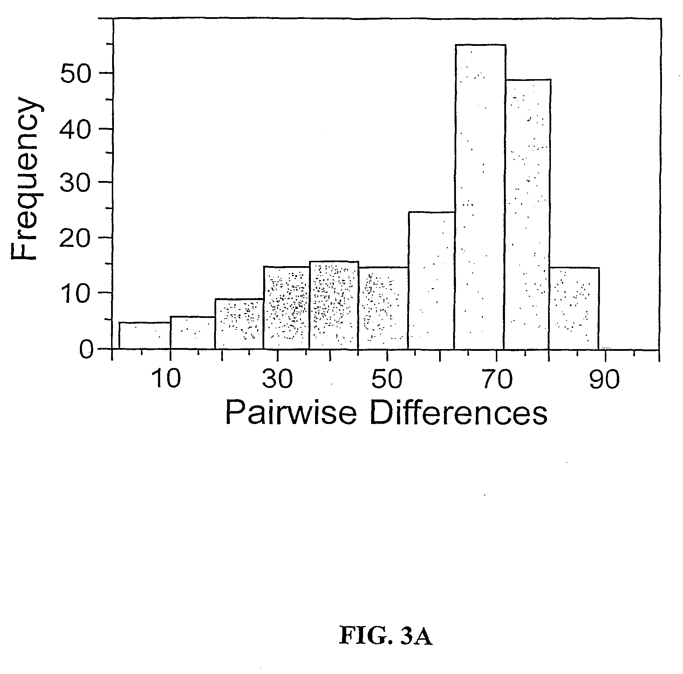

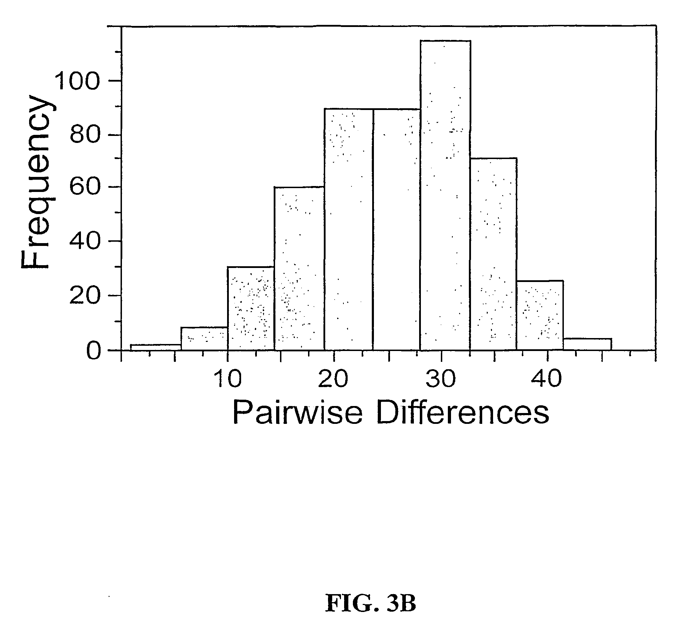

Figure 3 shows mismatch distributions of pairwise nucleotide differences between 53 mtDNA genomes (excluding the D-loop) for: a) African and; b) non-African individual.

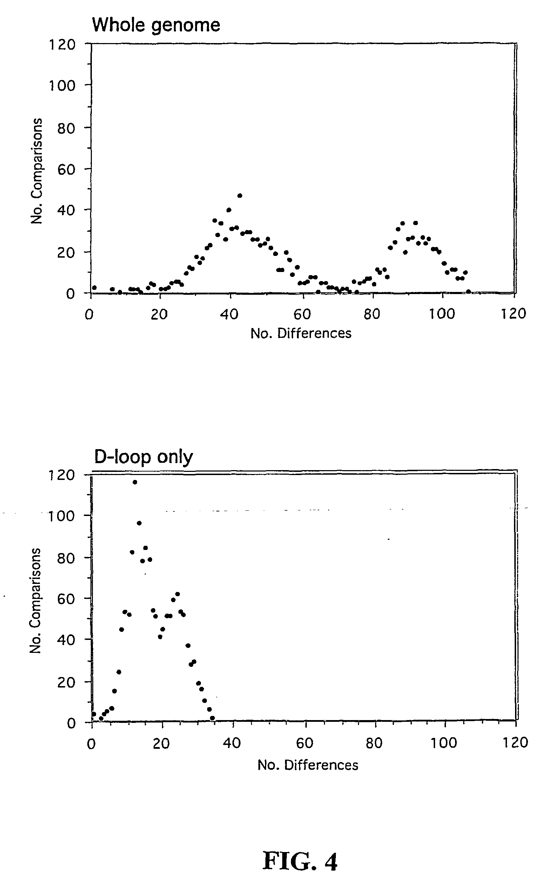

Figure 4 shows distribution in the number of differences in pairwise comparisons of 53 sequences for the D-loop only and for the complete mtDNA sequence.

### Identification of genetic variation in the human mitochondrial genome

To assess the global genetic diversity in human mtDNA we have determined the complete mtDNA genome of a total of 124 individuals. Individuals were selected as to cover most of the genetic diversity in the human species.

The complete mtDNA genome was amplified in segments using PCR and the fragments sequenced. The primers used for PCR amplification were as described by Reider et al (1998). Sequencing was performed on the PCR products directly using BigDye (Applied Biosystems) chemistry. Separation of sequencing ladders was performed on the ABI 377 instrument for automated fragment analysis. Both forward and reverse strands were sequenced. Sequence analysis was performed using Sequencing Analysis 3.3 (Applied Biosystems) and sequence alignment was made with Sequencher 3.1.1 (Gene Codes).

All the 124 complete mtDNA sequences are unique. A total of 1122 polymorphisms were identified among these 124 individuals. A list of the sites, the types of alternative nucleotides found at each of these sites, and the frequency of these different nucleotides is shown in Table. 1.

Our study of the entire mitochondrial genome has significant distinctions from previous studies of the D-loop. Most importantly, the sequences outside of the D-loop evolve in an approximately 'clock-like' manner, enabling a more accurate measure of mutation rate, and therefore improved estimates of times to evolutionary events (Ingman et al. 2000). The difference between the D-loop and the remaining molecule is visually evident in the contrast between the jumbled arrangement of polymorphic sites in the D-loop and the clear haplotypes defined by the sites in the rest of the molecule (Fig. 1). The Neighbor-Joining tree constructed from our mtDNA sequences has a strongly supported basal branching pattern (Fig. 2). The three deepest branches lead exclusively to sub-Saharan mtDNAs, with the fourth branch containing both Africans and non-Africans. The deepest, statistically supported branch (NJ bootstrap = 100) provides compelling evidence of a human mtDNA origin in Africa. Our data shows that the genetic variation (polymorphism) in the mtDNA genome, outside the D-loop is subject to very little parallellism (i.e. very few independent substitutions in multiple mtDNA lineages at the same site), increasing the ability to determine the maternal lineage and deducing the geographic and population origin based on this information. while the D-loop has too many parallel substitutions at numerous sites to be useful for this purpose.

### Utility of complete mtDNA genome variation for studies of human origin

Our analysis of the complete mitochondrial DNA genome excluding the D-loop have resulted in the following:
1) A very large number of novel polymorphisms outside the D-loop have been detected, many of which show a strong population-specific distribution. This increases the ability to determine the specific maternal lineage for an individual and define the geographic and population origin of an individual based on a biological sample.
2) A large database of complete mitochondrial genomes against which the genetic information from an individual can be compared, and geographic and population origin can be evaluated. For example, our database gives us an ability to identify with a very high probability whether the sample is obtained from an individual of African or Caucasian origin.

### Kit for the analysis of a subset of mtDNA polymorphisms detected

The variation in mtDNA between individuals has been studied mainly using Sanger sequencing. Pyrosequencing is in comparison to many other techniques for genetic typing very quick, robust and easy to use (Ronaghi et al. 1996).

The kit developed for mtDNA determination in the present invention is based on the analysis of 10 PCR fragments covering highly informative sites in the entire mitochondrial genome (Table 2). The system is based on the analysis of 19 pyrosequencing reactions, including 4 HVI, 4 HVII and 11 coding region reactions. This allows the analysis of some of the most informative regions of the entire mtDNA. The method enables the analysis of the D-loop and the coding regions of the mtDNA and can be used on a wide range of biological materials.

The analysis of the mitochondrial D-loop is performed from two separate PCR fragments. One hypervariable region I (HVI) fragment, which is analyzed in four separate pyrosequencing reactions. Similarly, the hypervariable region II (HVII) fragment is analyzed in four separate pyrosequencing reactions. In order to evaluate the technique, mitochondrial DNA (mtDNA) from a number of control samples have been analyzed. In total, 190 samples were analyzed for HVII, 120 samples were analyzed for HVI and finally 50 forensic forensic evidence materials were studied. The results were identical with sequencing data of the D-loop for the same individuals.

Pyrosequencing was further used to sequence 11 mtDNA coding regions in 36, previously sequenced, control samples. The 11 regions are chosen to cover the most informative sites throughout the entire mitochondrial genome using the previously generated complete mtDNA genome sequences. The results obtained using pyrosequencing were 100% identical to those obtained by the Sanger sequencing method.

To evaluate the system on limited amounts of DNA 50 biological samples with small amounts of DNA were analyzed for HVI and HVII by pyrosequencing. Among the materials analysed were samples from robber hoods, wigs, moustaches, shoes, cellular phones, watches, knives and guns. The pyrosequencing results were, for all the tested samples, identical to the results obtained using Sanger sequencing.

Combining the results of the eight HVI and HVII pyrosequencing reactions cover altogether approximately 88% (396/448) of the nucleotides after manual editing of the D-loop sequences. Complete Sanger sequencing of the D-loop gives a HVII fragment of 359 nucleotides and a HVI fragment of 403 nucleotides using the primer pairs L048/H408 and L15997/R16401 (Wilson et al. 1995). The pyrosequencing method covers 62% (222/359) of the nucleotides of the HVII fragment determined by Sanger sequencing and 56% (226/403) of the nucleotides of the HVI fragment determined by Sanger sequencing. In total, this gives a 59% coverage (448/762) of the D-loop determined by Sanger sequencing.

### EXPERIMENTAL SECTION

### Methodology for determining the complete mtDNA genome

### The PCR

The mitochondrial genome is amplified in 24 overlapping fragments by the PCR technique, using 24 primer pairs, as described by Reider et al (1998). Both the forward and reverse strands of these 24 DNA templates are sequenced using established techniques for DNA sequencing such as theBigDye Primer Cycle Sequencing Ready Reaction (Applied Biosystems) kits diluted 1:1 with 1x sequencing buffer, using the following components 1uL of DNA template
4uL reaction mix (2uL kit and 2uL 1x sequencing buffer)
Extension reactions in MJ Research Tetrad thermocycler with programs as follows:

### Forward reactions

| (ramping at 1°/second) |
|---|
| 96° 10 seconds |
| 55° 5 seconds |
| 70° 1 minute |
| <14 cycles> |
| 96° 10 seconds |
| 70° 1 minute |
| < 14 cycles> |
| Hold at 4° |

### Reverse Reactions

| (ramping at 1 °/ second) |
|---|
| 96° 10 seconds |
| 48° 5 seconds |
| 70° 1 minute |
| < 19 cycles> |
| 96° 10 seconds |
| 70° 1 minute |
| < 14 cycles> |
| Hold at 4° |

The extension products from these reactions are precipitated in 95% ethanol and the products spun down in a centrifuge at maximum speed for 25 minutes before removing the ethanol and allowing the pellets to air dry.

The pellets are rehydrated in a bromothymol loading buffer and loaded on an ABI 377 instrument for separation of sequencing ladders. A 96 well format was used allowing all the forward and reverse strands of 2 genomes to be run simultaneously. Sequencing gel analysis was performed using Sequencing Analysis 3.3 (Applied Biosystems) and the contigs were assembled into complete double stranded genomes with Sequencher 3.1.1 (Gene Codes). To aid in error checking, these completed genomes were then aligned with a consensus genome sequence of all the previously sequenced mtDNAs and all differences checked against the chromatograms for the new sequence. The new genome sequence could then be exported to the sequence database for analysis.

### Sequence Analysis

The new complete mtDNA genome sequence is aligned with the other sequences in the reference database. Pairwise numbers of differences can be calculated against every sequence in the database, for instance using a computer program such as PAUP*. The resulting distance matrix is a list of the number of differences between the sequence under analysis and each reference sequence. From this information, the number of matches with 0, 1 & 2 differences between an individual sequence and those in the database can be can be calculated. In our database of 124 complete mtDNA sequences, the number of variable sites when considering the D-loop sequences only is much lower than that for the complete sequence.

### Methodology and reagents for the kit assaying the mtDNA polymorphisms detected

### DNA preparations

Human DNA was extracted from PBLs of 190 Swedish blood donors, to serve as control samples. The forensic evidence material was purified by one of three methods. The Wizard Genomic DNA Extraction Kit (Promega) was used to extract DNA from most of the evidence material collected by cotton swabs and also from blood. Chelex 100 (Bio-Rad) was used to extract DNA from bloodstains. The method has been developed for extracting DNA from forensic-type samples for use with PCR (Walsh et al. 1991). Hair samples were extracted with an extraction procedure that uses proteinase K and DTT (Vigilant 1999).

### Primer design and PCR

The software Primer Express^{®} (Applied Biosystems) was used for PCR and sequence primer design. An optimal selection has been made to cover most of the informative polymorphism in the D-loop, where hypervariable region I and II (HVI and HVII) are PCR amplified in two different PCR products (Table 2). The coding region polymorphisms are amplified in 11 separate reactions and are analysed in 11 pyrosequencing reactions, using the forward PCR primer as sequencing primer. PCR amplification for control samples was set up in 70 µl containing 1.5 µl DNA, 200 nM of a normal and a biotinylated primer (Table 2), 200 nM of each dNTP, 1.5 mM MgCl₂, 2 U AmpliTaq^{®} Gold DNA Polymerase and 1 × GeneAmp^{®} PCR Buffer II. The PCR amplification for the forensic evidence material was set up in 100 µl with 10 µl DNA, 200 nM of the normal and the biotinylated primer, 200 nM of each dNTP, 2.4 mM MgCl₂, 10 UAmpliTaq^{®} Gold DNA Polymerase, 1.2x GeneAmp^{®} PCR Buffer II, 0.16 mg/ml BSA and 10 % glycerol. The amplifications were performed in an ABI 9600 instrument (Applied Biosystems). The samples were kept for 10 min at 95°C followed by 45 cycles of 30 sec at 95°C, 45 sec at 60°C (53°C for the coding region primers) and 60 sec at 72°C. The final extension was lengthened to 7 min. Tubes that contained all PCR components, but without template (NTC), were used to ensure that the reagents were free of contamination.

### Template preparation and pyrosequencing reaction

Streptavidin-coated beads were used as a solid phase support to obtain single-stranded biotinylated PCR products, as described by Pyrosequencing AB. Some reactions required 440 ng of Single Stranded DNA Binding Protein (SSB) (Amersham Pharmacia Biotech) added to the primed DNA template, prior to pyrosequencing (Ronaghi 2000). The sequencing was performed at room temperature and 15 µl of the 70 µl PCR reaction was used with 400 nM sequence primer. Enzyme and substrate mixture (prototype of PSQ^{™} 96 SQA Reagent Kit) were added to all samples in a PSQ^{™} 96 System (Pyrosequencing AB) with a prototype of PSQ^{™} 96 SQA Software (sample entry, instrument control and evaluation). The procedure was carried out by stepwise elongation of the primer strand during sequential dispensation of different dNTPs (AαS, C, G and T) followed by degradation of nucleotides. Optimal cyclic dispensation orders were chosen for each fragment. The sequences were edited and compared with Anderson *et al.* reference standard (Anderson et al. 1981). The control samples were sequenced using an ABI 377 instrument and BigDye Terminator chemistry.

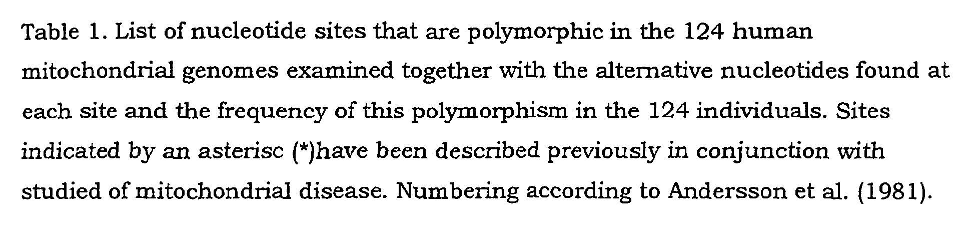

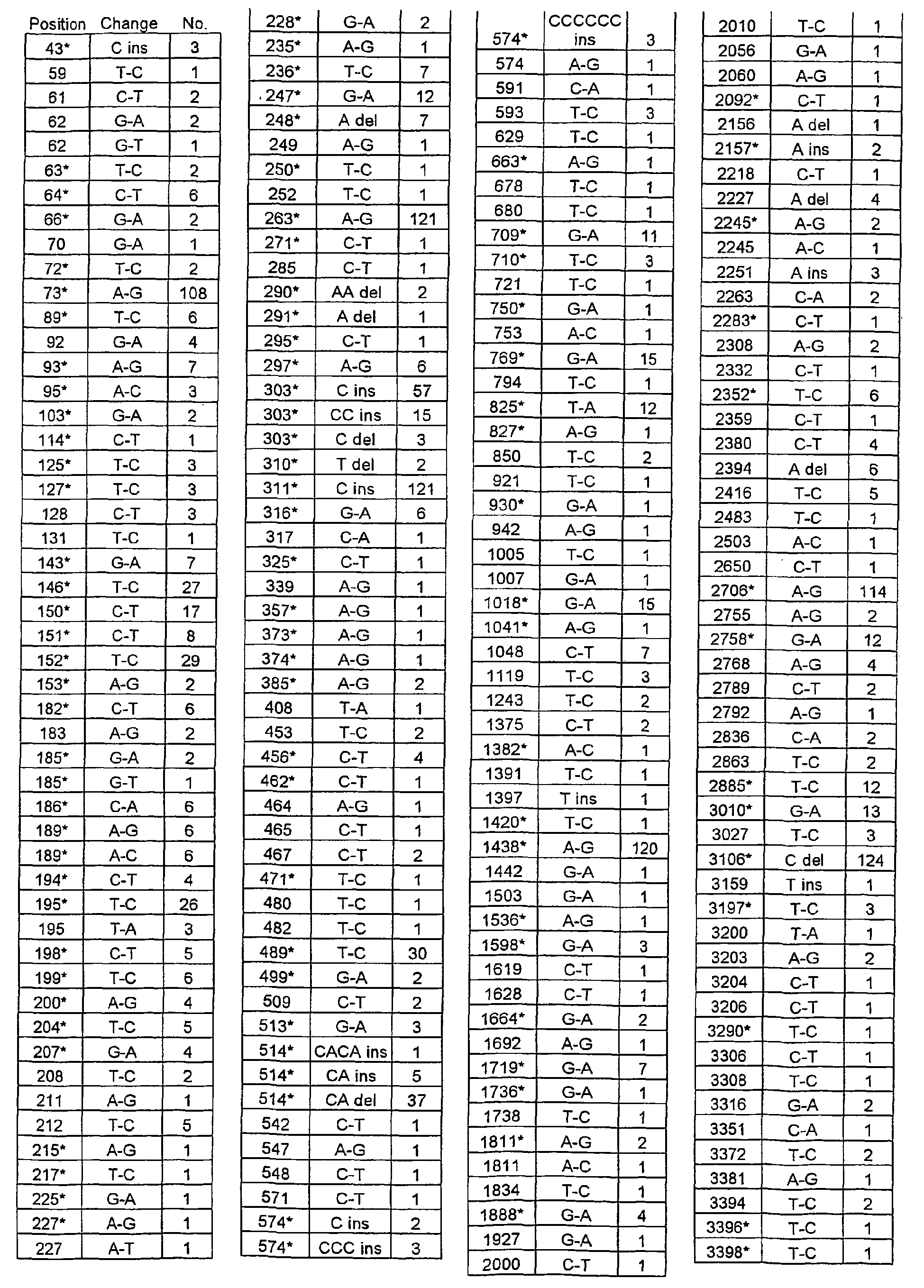

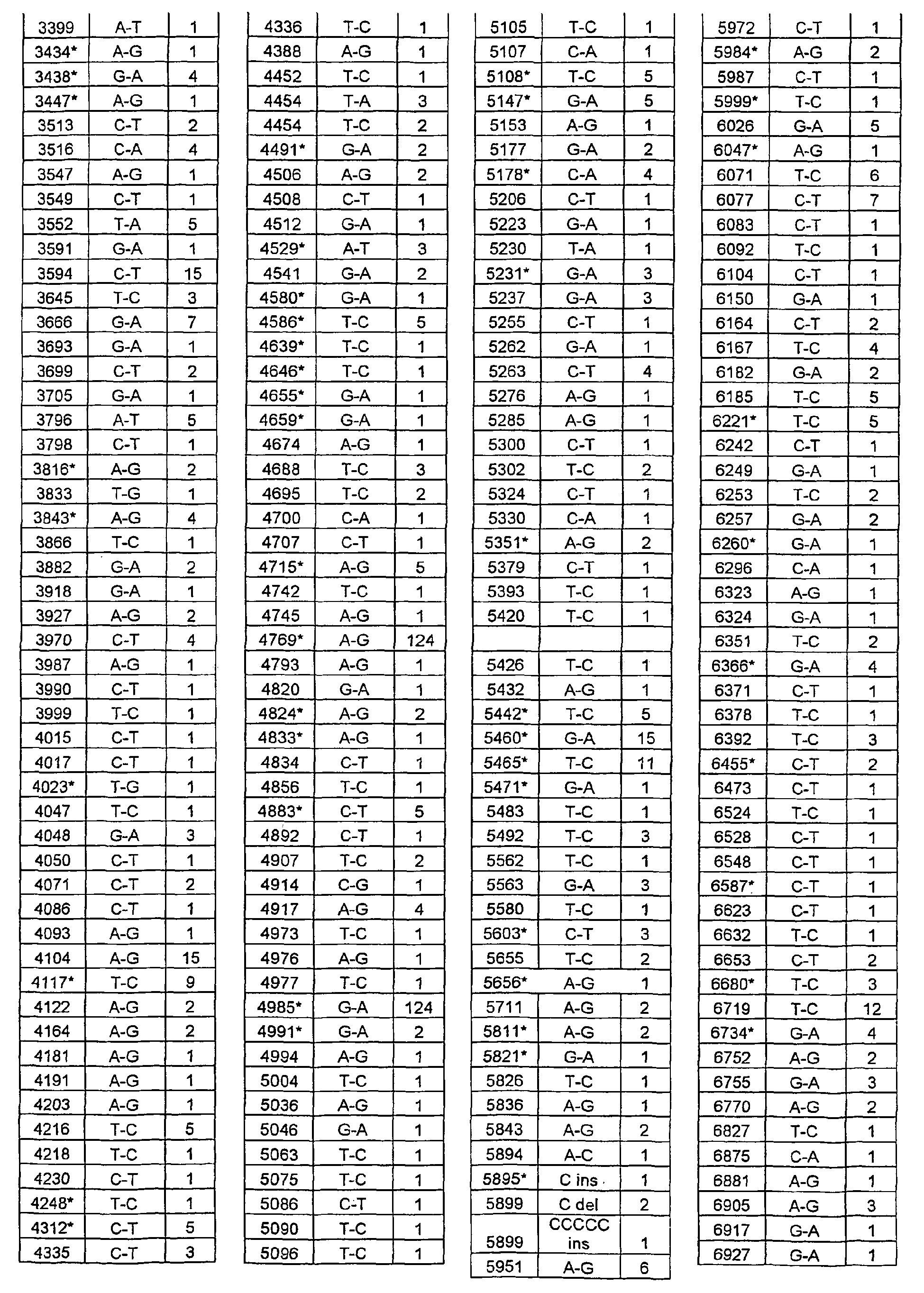

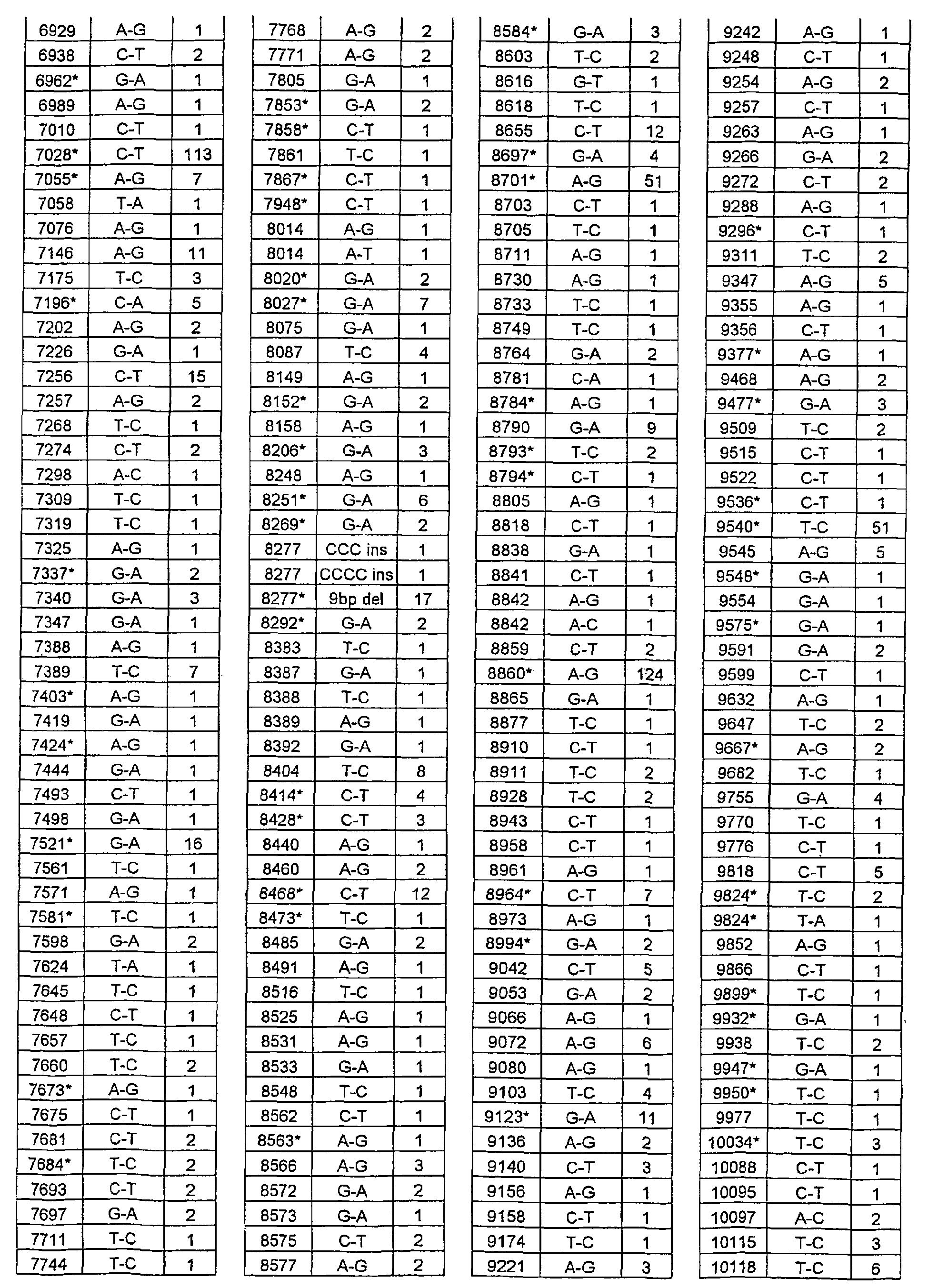

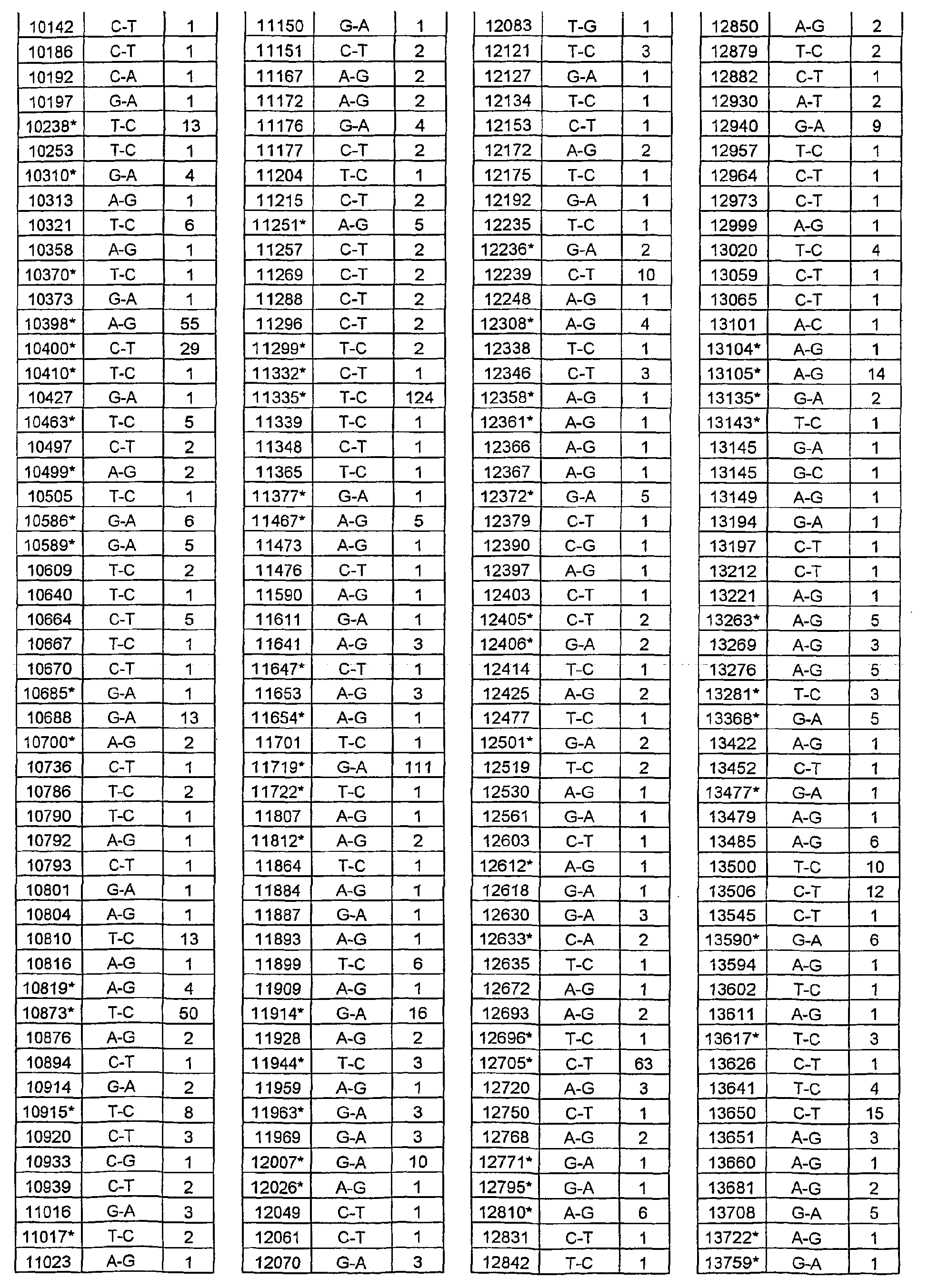

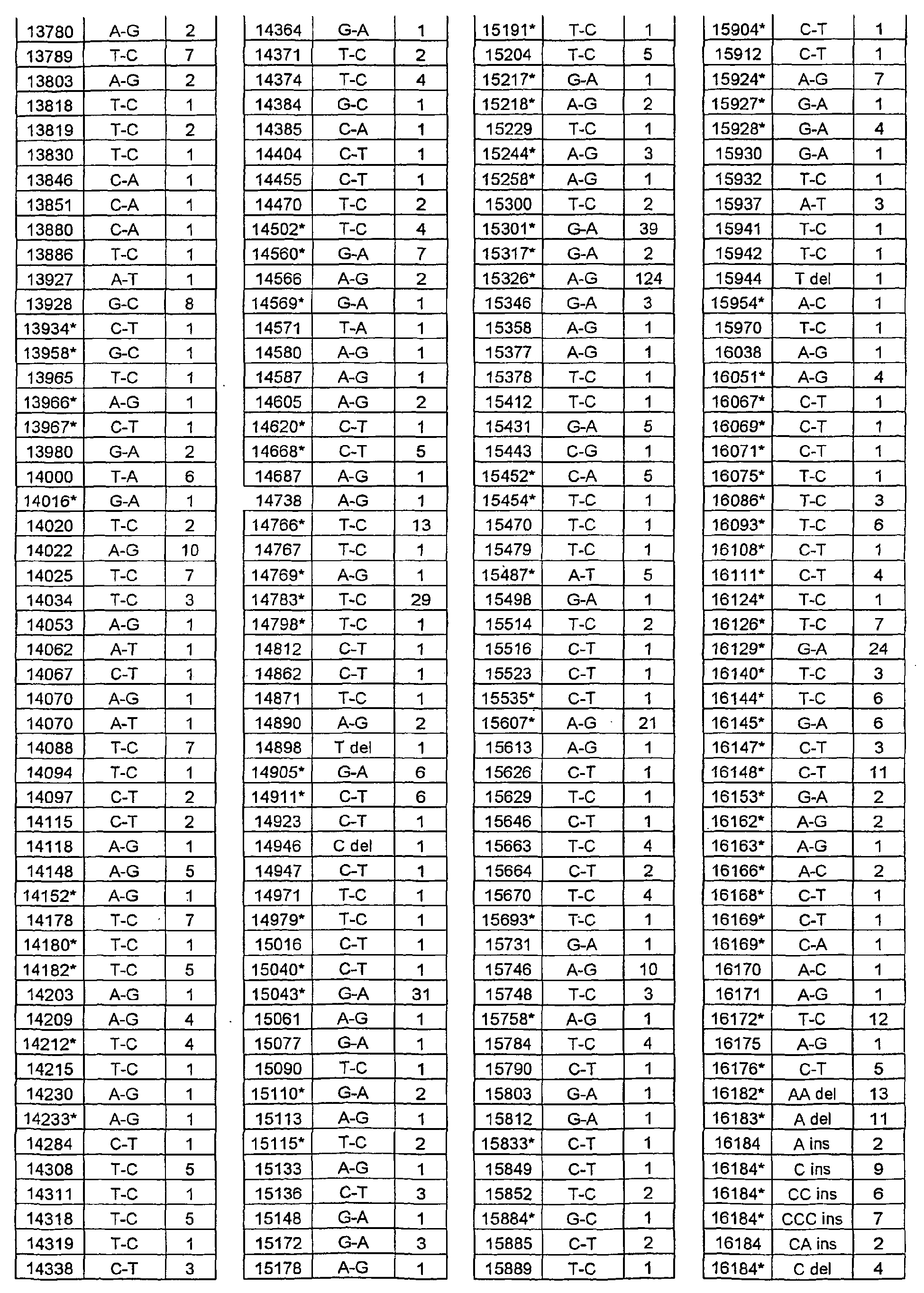

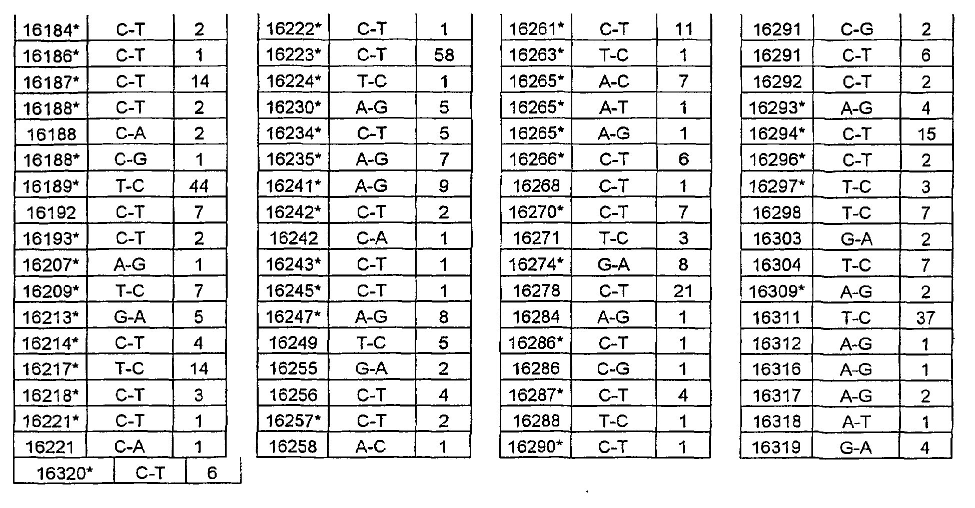

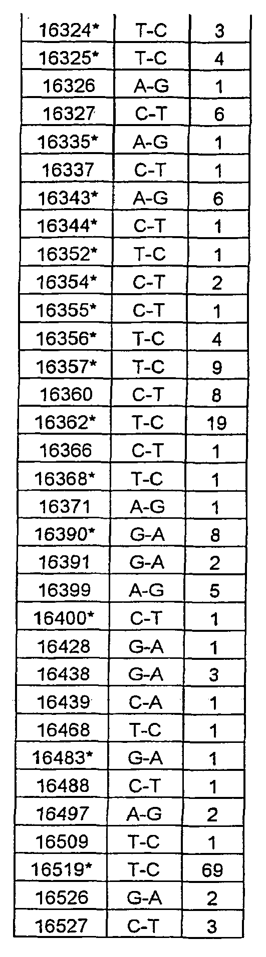

**Table 2. PCR and sequencing primers used for the analysis of mtDNA polymorphism**

| Primer Name* | PCR Primer | Sequene Primer | Tₘ (°C) | Sequence (5'-3') | Dispensation order | SSB Required |
|---|---|---|---|---|---|---|
| II 45 | X | X | 51,3 | ATG CAT TTG GTA TTT TGG TCT G | TCGA | |
| II 111 | | X | 43,3 | ACC CTA TGT CGC AGT ATC T | TCGA | |
| II 162 | | X | 50,1 | CGC ACC TAC GTT CAA TAT TAC A | CTGA | |
| II 216 | | X | 44,3 | TTA ATG CTT GTA GGA CAT AAT AA | CTGA | |
| II 287 B | X | | 51,2 | TTG TTA TGA TGT CTG TGT GGA AAG | | |
| C 431 | X | X | 53,6 | CAC CCC CCA ACT AAC ACA | ACGT | |
| C 637 B | X | | 53,1 | GTG ATG TGA GCC CGT CTA A | | |
| C 2988 | X | X | 51,8 | CGA TGT TGG ATC AGG ACA | ACGT | |
| C 3216 B | X | | 52,4 | GG TGG GTG TGG GTA TAA | | |
| C 3403 | X | X | 56,8 | CTA CGC AAA GGC CCC AA | CAGT | X |
| C 3641 B | X | | 56 | GCT AGG CTA GAG GTG GCT AGA A | | |
| C 4156 | X | X | 52,5 | CAA CTC ATA CAC CTC CTA TGA AA | ACGT | |
| C 4367 B | X | | 52,6 | TTG GAT TCT CAG GGA TGG | | |
| C 4882 | X | X | 49 | CCA TCT CAA TCA TAT ACC AAA | ATGC | |
| C 5138 B | X | | 50 | GGA GTT TAA GTT GAG TAG TAG GAA | | |
| C 8665 | X | X | 52 | CAA TGA CTA ATC AAA CTA ACC TCA | ATGC | |
| C 8803 B | X | | 51,1 | TAA ATG AGT GAG GCA GGA GT | | |
| C 12346 | X | X | 47,9 | CAC ACT ACT ATA ACC ACC CTA A | ACGT | X |
| C 12541 B | X | | 49,1 | CTC AGT GTC AGT TCG AGA TAA | | |
| C 12673 | X | X | 45,7 | AAC ATT AAT CAG TTC TTC AAA | ACGT | X |
| C 12861 B | X | | 46,7 | GTT GTA TAG GAT TGC TTG AA | | |
| C 14747 | X | X | 53,4 | ATG ACC CCA ATA CGC AAA | ACGT | X |
| C 14949 B | X | | 54,1 | TGG GCG ATT GAT GAA AAG | | |
| C 15883 | X | X | 50,2 | GGC CTG TCC TTG TAG TAT AAA | ACGT | |
| C 16083 B | X | | 52 | GGT TGT TGA TGG GTG AGT C | | |
| C 16496 | X | X | 48,5 | GAC ATC TGG TTC CTA CTT CA | ACGT | X |
| C149B | X | | 47,7 | ATG AGG CAG GAA TCA AA | | |
| I 16105 | X | X | 50,2 | TGC CAG CCA CCA TGA ATA | CTGA | X |
| I 16168 | | X | 45,1 | CCA ATC CAC ATC AAA ACC | CTGA | X |
| I 16203 | | X | 40,4 | AGC AAG TAC AGC AAT CAA | CTGA | |
| I 16266 | | X | 42,1 | CCC ACT AGG ATA CCA ACA | CTGA | |
| I 16348 B | X | | 51,8 | GAC TGT AAT GTG CTA TGT ACG GTA AA | | |

| | | | | | | |
|---|---|---|---|---|---|---|
| *I = HVI, II = HVII, C = coding region, B = biotin labeled reverse primer. | | | | | | |

### References

Allen, M. Engström, AS. Meyers, S. Handt, O. Saldeen, T. von Haeseler, A. Paabo, S. Gyllensten, U. 1998. Mitochondrial DNA sequencing of shed hairs and saliva on robbery caps: sensitivity and matching probabilities. Journal of Forensic Science 43(3):453-64.
Anderson, S., A. T. Bankier, et al. (1981). Sequence and organization of the human mitochondrial genome. Nature 290(5806): 457-65.
Bodenhagen D, Clayton DA. The number of mitochondrial deoxyribonucleic acid genomes in mouse L and human HeLa cells. J Biol Chem 1974;249:7991-5.
Brown WM, Prager EM, Wang A, Wilson AC. Mitochondrial DNA sequences of primates: tempo and mode of evolution. J. Mol Evol 1982;18: 225-39.
Gill P, Ivanov PL, Kimpton C, Piercy R, Benson N, Tully G, et al. Identification of the remains of the Romanov family by DNA analysis. Nat Genet 1994;6:130-5.
Ginther C, Issel-Tarver L, King MC. Identifying individuals by sequencing mitochondrial DNA from teeth. Nat Genet 1992;2:135-8.
Hagelberg E, Sykes B. Ancient bone DNA amplified. Nature 1989;342:485.
Handt O, Richards M, Trommsdorff M, Kilger C, Simanainen J, Georgiev O, et al. Molecular genetic analyses of the Tyrolean ice man. Science 1994;264:17758.
Higuchi R, von Berolding CH, Sensabaugh GF., Erlich HA. DNA typing from single hairs, Nature, 1988;332:543-6.
Holland MM, Fisher DL, Mitchell LG, Rodriquez WC, Canik JJ, Merril CR, et al. Mitochondrial DNA sequence analysis of human skeletal remains: Identification of remains from the Vietnam war. J For Sci 1993;38:542-53.
Hutchison III CA, Newbold JE, Potter SS, Edgell M. Maternal inheritance of mammalian mitochondrial DNA. Nature 1974;251:536-538.
Ingman, M., H. Kaessmann, et al. (2000). "Mitochondrial genome variation and the origin of modern humans." Nature 408(6813): 708-13.
Pääbo S, Higuchi RG, Wilson AC. Ancient DNA and the polymerase chain reaction J Biol Chem 1989;264:9709-12.
Reider, MJ Taylor, SL, Tobe, VO & Nickerson, DA. Automating the identification of DNA variations using quality-based fluorescence re-sequencing: analysis of the human mitochondrial genome. Nucleic Acids Res. 26, 967-973 (1998).
Ronaghi, M., S. Karamohamed, et al. (1996). "Real-time DNA sequencing using detection of pyrophosphate release." Anal Biochem 242(1): 84-9.
Stoneking M, Hedgecook D, Higuchi RG, Vigilant L, Erlich HA. Population variation of human mtDNA control region sequences detected by enzymatic amplification and sequence-specific oligonucleotide probes. Am J Hum Genet 1991;48: 370-82.
Vigilant, L. (1999). "An evaluation of techniques for the extraction and amplification of DNA from naturally shed hairs." Biol Chem 380(11): 1329-31.
Walsh, P. S., D. A. Metzger, et al. (1991). "Chelex 100 as a medium for simple extraction of DNA for PCR-based typing from forensic material." Biotechniques 10 (4): 506-13.
Wilson, M. R., D. Polanskey, et al. (1995). "Extraction, PCR amplification and sequencing of mitochondrial DNA from human hair shafts." Biotechniques 18(4): 662-9.
Wilson MR, Holland MM, Stoneking M, DiZinno, JA, Budowle B. Guidelines for the use of mitochondrial DNA sequencing in forensic medicine. Crime Lab Dig 1993;20:68-77.

## Claims

1. A method for determining the origin or identity of a human with high accuracy comprising the following steps:
a) determining polymorphic sites in the nucleic acid sequence of the complete mitochondrial genome outside the D-loop in a sample from a human subject who's origin or identity is to be determined, wherein the human mitochondrial nucleic acid sequences is determined by DNA sequencing, or in the case of genetic markers, on assays such as enzymatic ligation assays (OLA, padlock) enzymatic cleavage assays (Taqman), enzymatic extension assays (minisequencing, pyrosequencing) or other assays for typing of genetic polymorphisms; and
b) relating the information from step a) to mitochondrial nucleic acid sequence information of known origin.

2. A method according to claim 1, wherein the known information in step b) is derived from a database of nucleic acid sequence information of the complete mitochondrial genome from humans of diverse origin.

3. A method according to claim 1, wherein the known information in step b) comprises the polymorphic sites mentioned in Table 1.

4. A method according to any of the above claims, wherein the mitochondrial nucleic acid sequence is determined by pyrosequencing.

5. A method according to any of the above claims, wherein the means for analysis are selected from the reagents listed in Table 2 of the present patent application.

6. A kit for determining origin or identity of a human with high accuracy, comprising means for analysis selected from reagents listed in Table 2 covering informative sites outside the D-loop of the mitochondrial genome.

7. A kit according to claim 6, wherein the means for analysis are pyrosequencing means.

## Patentansprüche

1. Verfahren zur Bestimmung der Herkunft oder der Identität eines Menschen mit hoher Genauigkeit, umfassend die folgenden Schritte:
(a) Bestimmen polymorpher Stellen in der Nukleinsäuresequenz des vollständigen Mitochondriengenoms außerhalb des D-Loops in einer Probe einer Testperson, deren Herkunft oder deren Identität bestimmt werden soll, wobei die humanen mitochondrialen Nukleinsäuresequenzen mittels DNA-Sequenzieren bestimmt werden, oder im Fall genetischer Marker mittels Assays wie enzymatischer Ligationsassays (OLA, padlock), enzymatischer Spaltungsassays (Taqman), enzymatischer Extensionsassays (Minisequencing, Pyrosequencing) oder anderer Assays für den Nachweis genetischer Polymorphismen; und
(b) Vergleichen der Information aus Schritt (a) mit mitochondrialer Nukleinsäuresequenzinformation bekannten Ursprungs.

2. Das Verfahren gemäß Anspruch 1, wobei die bekannte Information von Schritt (b) aus einer Datenbank von Nukleinsäuresequenzinformation des vollständigen mitochondrialen Genoms von Menschen verschiedener Herkunft stammt.

3. Das Verfahren gemäß Anspruch 1, wobei die bekannte Information in Schritt (b) die polymorphen Stellen von Tabelle 1 umfasst.

4. Das Verfahren gemäß einem der vorangehenden Ansprüche, wobei die mitochondriale Nukleinsäuresequenz mittels Pyrosequencing bestimmt wird.

5. Das Verfahren gemäß einem der vorangehenden Ansprüche, wobei die Analysemittel ausgewählt sind aus den Reagenzien von Tabelle 2.

6. Kit zur Bestimmung der Herkunft oder der Identität eines Menschen mit hoher Genauigkeit, umfassend Analysemittel ausgewählt aus den Reagenzien von Tabelle 2, die informative Bereiche außerhalb des D-Loops des mitochondrialen Genoms abdecken.

7. Der Kit gemäß Anspruch 6, wobei die Analysemittel Mittel für das Pyrosequencing sind.

## Revendications

1. Procédé permettant la détermination de l'origine ou de l'identité d'un être humain avec une précision élevée comprenant les étapes suivantes :
a) détermination des sites polymorphiques dans la séquence d'acide nucléique du génome mitochondrial complet à l'extérieur de la boucle D dans un échantillon provenant d'un sujet humain dont l'origine ou l'identité doit être déterminée, dans laquelle la séquence d'acide nucléique mitochondrial humain est déterminée par séquençage d'ADN ou bien, dans le cas de marqueurs génétiques, lors d'essais tels que des essais par ligation enzymatique (OLA, Padlock), des essais par clivage enzymatique (Taqman), des essais par extension enzymatique (miniséquençage, pyroséquençage) ou d'autres essais pour le génotypage de polymorphismes génétiques ; et
b) liaison des informations provenant de l'étape a) avec des informations concernant une séquence d'acide nucléique mitochondrial d'origine connue.

2. Procédé selon la revendication 1, dans lequel les informations connues à l'étape b) proviennent d'une base de données d'informations sur la séquence d'acide nucléique du génome mitochondrial complet d'êtres humains d'origines diverses.

3. Procédé selon la revendication 1, dans lequel les informations connues à l'étape b) comprennent les sites polymorphiques mentionnés dans le Tableau 1.

4. Procédé selon l'une des revendications précédentes, dans lequel la séquence d'acide nucléique mitochondrial est déterminée par pyroséquençage.

5. Procédé selon l'une des revendications précédentes, dans lequel les moyens d'analyse sont sélectionnés parmi les réactifs listés dans le Tableau 2 de la présente demande de brevet.

6. Kit permettant de déterminer l'origine ou l'identité d'un être humain avec une précision élevée, comprenant des moyens d'analyse sélectionnés parmi les réactifs listés dans le Tableau 2 qui concerne des sites informatifs à l'extérieur de la boucle D du génome mitochondrial.

7. Kit selon la revendication 6, dans lequel les moyens d'analyse sont des moyens de pyroséquençage.
